Europäisches Patentamt

European Patent Office

Office européen des brevets

(11) Publication number: **0 278 591**
**A1**

## (12) EUROPEAN PATENT APPLICATION

(21) Application number: 88300159.6

(22) Date of filing: 11.01.88

(51) Int. Cl.4: **A61F 5/37** , B60R 22/10

(30) Priority: 12.01.87 US 2212
30.06.87 US 68020

(43) Date of publication of application:
17.08.88 Bulletin 88/33

(84) Designated Contracting States:
DE ES FR GB IT

(71) Applicant: **Daniels, Jerry L.**

**Ooltewah Tennessee(US)**

(72) Inventor: **Daniels, Jerry L.**

**Ooltewah Tennessee(US)**

(74) Representative: **Funge, Harry et al**
**M'CAW & CO. 41-51 Royal Exchange Cross**
**Street**
**Manchester M2 7BD(GB)**

(54) **Prisoner leg restraint.**

(57) A system for restraining the lower limbs of a prisoner being conveyed in a vehicle, such as a police car, to preclude injury to persons and to property by violent kicking action of the prisoner. The system utilizes a laminated strap (18) having synthetic hook and loop fastening elements formed on one ply of the laminate and a reinforced vinyl backing (28) formed on the other ply. One end of the strap is fastened to a grommet (30) through which the other end of the strap may be drawn and folded over so that hook elements (20) and loop elements (22) may be engaged to adhere while the vinyl portion of the strap is tightly engaged about the lower limbs of the prisoner. In one embodiment, a belt (36) having a hook (38) including a biased latch (50) at one end of the belt is attached to the strap at the other end, and the hook can be secured to an anchor on the floor of the vehicle. In a second embodiment, the belt has a catch in the form of a buckle at one end of the belt and is attached to the strap at the other end, and the catch may be tossed out the door of the vehicle and become lodged against a portion of the door and the adjacent door frame. The catch may have a magnet bonded to a flat surface thereof so that it can adhere to the vehicle and not fall away. The attachment of the belt to the strap may be either by a fixed securement or by an adjustable connection.

FIG. I

## PRISONER LEG RESTRAINT

### BACKGROUND OF THE INVENTION

This invention relates to the restraint of the lower limbs of a prisoner to a police vehicle and more particularly to a belting system utilizing a laminated fastening strap for encircling the lower limbs of a prisoner, the belting having connecting means for attachment to the floor of the vehicle or between the door and frame of the vehicle, and the laminated strap having synthetic material fastening elements which rapidly adhere when pushed together, the fastening elements being laminated to a backing of sufficient strength to restrain the limbs of a violent prisoner.

It is well known in the law enforcement field that substantial physical injury to an arresting officer and physical damage to public property has resulted when a suspect being taken prisoner resists arrest. It is notoriously well known to restrain the hands and arms of the prisoner by handcuffs or the like connecting the prisoner's hands together, in front of or behind his or her body. However, although the legs of the prisoner are powerful weapons, especially when the prisoner is emotionally charged, no leg restraining system has been developed which functions satisfactorily. Thus, there are many cases in which an arresting officer has been kicked violently as the prisoner is placed into a police cruiser, and also many more documented cases wherein the police cruiser or squad car has been badly battered by a prisoner's feet and legs as the prisoner is being driven to the station house or other detention center. In certain cases the entire partition between the police officer and the prisoner has been shattered, although most damage occurs to the doors, side panels and windows.

The use of shackles or chains to restrain the legs and feet of a prisoner is useful once a prisoner has been subdued, but such leg manacles cannot be readily placed on the prisoner and are not practical for use by an arresting officer. Moreover, unless heavily weighted they would not prevent the prisoner from lashing out with his or her feet.

Because of numerous instances in which police officers and public property have been damaged, violently acting prisoners have been restrained by other means resulting in charges of "police brutality" and, of course, costly litigation and negative publicity.

### SUMMARY OF THE INVENTION

Consequently, it is a primary object of the present invention to provide restraining means for the lower limbs of a prisoner taken into custody by law enforcement officials, the restraining means being rapidly and securely attached about the prisoner and readily secured to a police vehicle.

It is another object of the present invention to provide flexible belting including a laminated fastening strap which can securely and rapidly encircle and restrain the lower limbs such as the legs, thighs or ankles of a prisoner taken into police custody, the belting including means for rapidly connecting the strap to a relatively fixed surface, such as the floor of a police cruiser or the like, to preclude violent kicking and thrashing of the prisoner's feet.

It is a further object of the present invention to provide flexible belting including a laminated fastening strap which can securely and rapidly encircle and restrain the lower limbs such as the legs, thighs or ankles of a prisoner taken into police custody, the belting including means for rapidly attaching the strap to the exterior of a police cruiser or the like between the door and door frame, to preclude violent kicking and thrashing of the prisoner's feet.

It is a further object of the present invention to provide lower limb restraining means for resisting prisoners being conveyed by a police vehicle or the like to a detention center such as a station house or the like, the restraining means comprising a laminated strap having synthetic loop and fastener elements extending from a surface thereof and bonded to a strong plastic sheet of material such as reinforced vinyl, the strap being secured adjacent one end to a grommet through which the other end may be directed after encircling the lower limbs of the prisoner so that one type of fastener element may be superposed over and rapidly connected to fastener elements of the other type on the adjacent portion of the strap, the outer surfaces of the plastic sheet facing the limbs of the prisoner, and a second grommet carried by the strap for attaching one end of belting, the belting having a securing member at the other end for rapid attachment to the vehicle. The securing member may be a latch adapted to be connected to an anchor fixed to the floor of the conveyor, or it may be a catch adapted to be lodged between the door and door frame of the vehicle on the exterior thereof and preferably to adhere to the exterior surface once the belting has been tensioned to pull the catch against the vehicle.

Accordingly, the present invention provides a restraining belt means for the lower limbs of a prisoner or the like, the belt means comprising a laminated strap of the type forming the subject matter of my U.S. Patent No. 4,643,932 having first and second plies of material bonded together, the first ply having interconnectable plastic hook and loop fastener elements such as that sold under the trademark VELCRO bonded to a second ply of a strong pliable sheet of material such as reinforced vinyl. The hook and loop fastener elements extend from one surface of the strap so that one set of the elements may be folded back onto and adhere to the other set of elements when pressed together. One end of the strap is secured to a grommet or the like through which the other or free end of the strap may be drawn with the outer surface of the second ply folded upon itself in superposed relationship with the limbs of the prisoner therebetween. The free end of the strap may then be looped over the grommet for engaging one set of the fastener elements with the other set. One end of additional belt means is carried by the strap in either fixed relationship thereto or adjustable thereon preferably by means of a second grommet or the like, the other end of the additional belt means having either a latch adapted for rapid attachment to an anchor fixed to the vehicle within which the prisoner is to be restrained, or the additional belt means may have an enlargement or catch adapted to be trapped rapidly between the edge of a door and the door frame or doorjamb on the exterior of a police cruiser or the like within which the prisoner is to be restrained.

Thus, a law enforcement officer after restraining the arms of a prisoner by handcuffs or the like may restrain the legs of the prisoner by looping the strap about the legs or thighs and rapidly pressing the VELCRO fastener elements together, thereafter attaching the latch to the anchor, or merely toss the catch out the door of the prisoner compartment and rapidly slam the door shut. In the latter case a pull on the belt by the prisoner will then lodge the catch against the exterior of the vehicle.

BRIEF DESCRIPTION OF THE DRAWINGS

The particular features and advantages of the invention as well as other objects will become apparent from the following description taken in connection with the accompanying drawings, in which:

Fig. 1 is a perspective view depicting a prisoner having his lower limbs restrained by restraining belt means constructed in accordance with a first embodiment incorporating the principles of the present invention;

Fig. 2 is an elevational view of the restraining belt means of Fig. 1 with portions thereof broken away;

Fig. 3 is a perspective view of restraining belt means of Fig. 1, wherein the anchor attaching belting is fixedly secured to the strap;

Fig. 4 is a view similar to Fig. 3 illustrating a variation thereof wherein the anchor attaching belt means is adjustably disposed along the strap means;

Fig. 5 is a cross sectional view taken substantially along Fig. 5-5 of Fig. 3;

Fig. 6 is a perspective view depicting a prisoner within the prisoner compartment of a police vehicle or the like having his lower limbs restrained by restraining belt means constructed in accordance with a second embodiment of the present invention;

Fig. 7 is an exterior elevational view of a portion of the police vehicle illustrating the restraining catch of the restraining belt means of Fig. 6 at the outside of the vehicle;

Fig. 8 is a perspective view of one form of restraining belt means constructed in accordance with the second embodiment of the present invention; and

Fig. 9 is an enlarged perspective view of the catch of Figs. 7 and 8 lodged against the exterior surface of the vehicle.

DESCRIPTION OF THE PREFERRED EMBODIMENT

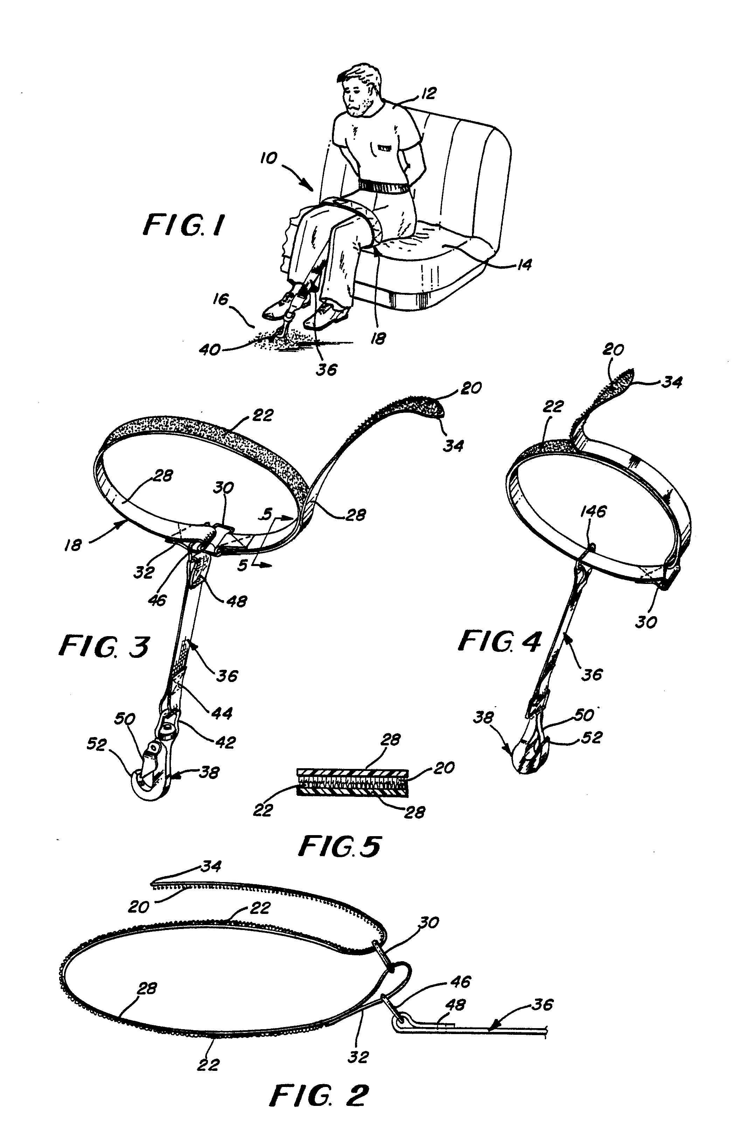

Referring now to the drawings and particularly to Fig. 1, a restraining system 10 constructed according to a first embodiment of the present invention is illustrated as applied to a prisoner 12 in lawful custody seated on a seat of an official vehicle while being conveyed to a police station or the like, the vehicle having a floor 16. As conventional with regard to those disposed to violence, the arms of the prisoner are handcuffed behind his back. Conventionally, the prisoner's lower limbs, such as his legs, are free and, if the prisoner is emotionally charged and highly violent, his feet can do great personal and physical damage. In some instances to minimize injuries the prisoner's shoes are removed. However, even if that can be accomplished by a single arresting officer without injury, for those prisoners trained in the martial arts even this is of minimal value. Thus, there has been a great need to provide law enforcement officials with the means for restraining the lower limbs of a prisoner when believed necessary. Such a restraining means must be easily and readily applied by an officer in the field and in the heat of making an arrest.

As illustrated in the drawings, the present invention provides a lower limb restraining means comprising a strap 18 formed from a laminate as described in the aforesaid U.S. Patent No. 4,643,932. On one surface of the laminate there are synthetic hook and loop fastener elements such as is known in the trade as "VELCRO" or similar material comprising a plastic sheet having a myriad of closely spaced synthetic plastic hooks 20 and loops 22 which when pushed or squeezed together interlock to form a strong connection which resist separation by a pull in the plane of the interacting parts, but which may be pulled apart by a separating pull on an end of one of the parts at an angle to the plane. A substantial longitudinally extending portion of the fastening surface of the strap preferably has the hook elements while the remainder of the strap carries the loop elements. The other surface of the laminate comprises a backing 28 preferably formed from reinforced vinyl, which is bonded along one surface to the rear surface of the hook and loop fastener elements, preferably along the borders of the strap, and preferably also at selected locations transverse to the borders, as described in the aforesaid U.S. Patent. Thus, a strap comprising two plies is provided wherein one of the plies comprises the "VELCRO" extending from a surface thereof and the other ply comprising a reinforced vinyl sheet, the reinforced vinyl and the bond between the two plies forming an extremely strong strap. The strap may be of any convenient length, and it has been found that a strap of approximately 45 inches in length has worked well for its intended purpose, and the length of the portion of the strap having the loops 22 was approximately 30 inches with the hooks 20 comprising at least a substantial portion of the remainder of the strap. Moreover, the strap may comprise a single length or may be formed from two laminated strips secured together, one of the strips having the hooks and the other having the loops.

As illustrated in various figures of the drawing, one and of the strap is looped about a grommet 30, folded back upon itself for a short distance as illustrated at 32 and secured to the superposed portion of the strap over which it is folded. The grommet preferably is a metal ring or any similar type member through which the strap may be drawn. Securement may be by stitching or the like. Although either the hook or the loop end of the strap may be attached to the grommet 30 as aforesaid, the preferred end is that which carries the loop 22, as illustrated. The free end 34, i.e., the end remote from the grommet 30, may then be drawn through the grommet and folded back such that the loops 22 are disposed in facing relationship with a portion of the hooks 20. Thus, the free end 34 of the strap is folded so that the vinyl surface of the ply at the loop end faces the vinyl surface at the grommet end of the strap The free end of the strap may thus be drawn through the grommet and folded over the grommet so that the interlinking hook and loop fastening elements of the "VELCRO" can be secured together. Accordingly, the strap may be encircled about the legs or thighs of a prisoner with only portions of the vinyl abutting the prisoner.

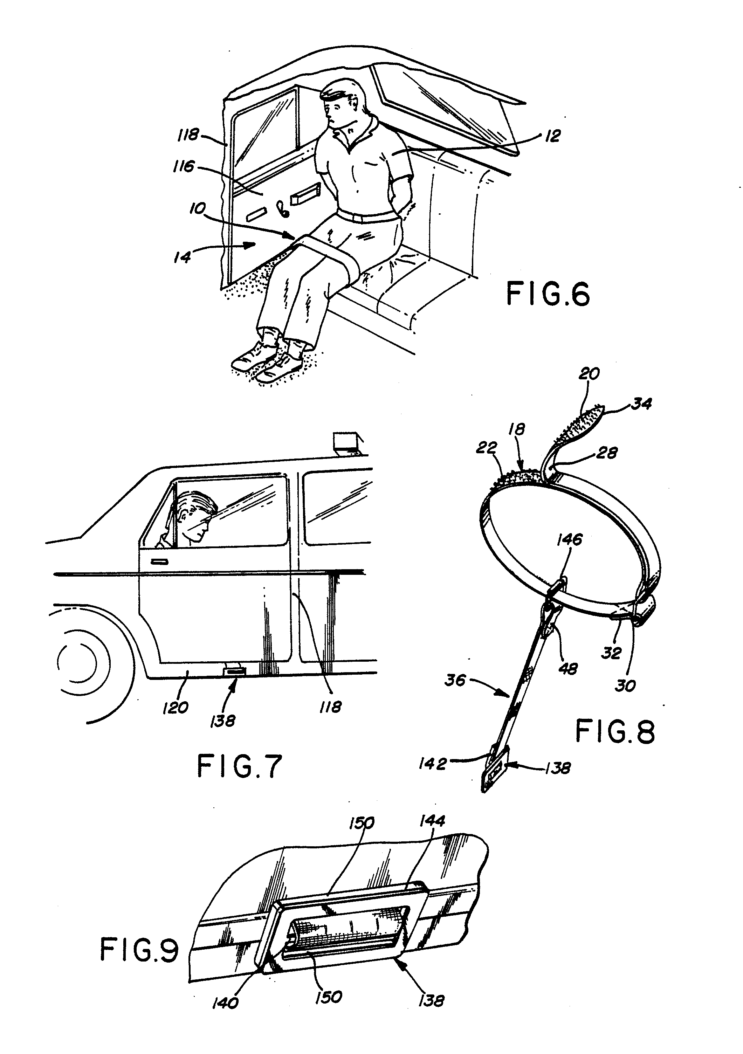

A second strap 36 having a hook member 38 secured adjacent one end thereof may be connected to the strap 18 at the other end, and the hook 38 secured to an anchor 40 on the floor of the vehicle or the like. The strap 36 may be of conventional belting or webbing such as utilized for automobile or airplane safety seat belts formed from woven nylon. One end of the belt 36 may thus be drawn through a ring 42 connected to the hook member 38 and folded back and sewn to the major portion of the belt 36 as illustrated at 44. The other end of the belt may either be fixedly or adjustably attached to the strap 18. For example, in Fig. 3 a second grommet 46 may be secured within the same loop of the strap 18 which secures the grommet 30, i.e., the lap 32 formed at the end of the strap remote from the free end 34. The belting 36 may then pass through the grommet 46 and be secured by stitching between the lap 48 and the adjacent superposed portion of the belt 36. With this construction the belt 36 and the hook 38 is always attached to the strap 18. In some instances it may be preferred that the belt 36 be adjustably attached to the strap 18. To this end, Fig. 4 illustrates that the belt 36 may be secured about a grommet 146 at the end remote from the hook 38 in the same manner as the belt 36 in Fig. 3 is fastened about the grommet 46, but the grommet 146 is not permanently attached to the strap 18 but is free to move therealong.

The hook 38, in the embodiments illustrated in Figs. 3 and 4, includes a resiliently biased latch member 50 which can be readily squeezed to overcome the bias and open the free end 52 of the hook so that it may receive or release the anchor 40, the bias being applied to urge the latch against the end 52 of the hook.

In the use of the apparatus of the first embodiment, after the prisoner is handcuffed, the strap 18 may be applied about his legs or thighs prior to or as he enters the vehicle. The hook 38 may then be secured to the anchor 40 thereby to restrain the prisoner from violently kicking his feet.

Although this device functions very well, there is the possibility that in the event of an accident involving the police cruiser, the prisoner could be inextricably trapped within the vehicle. It may therefore be desirable to devise a prisoner restraint of this type which can be secured and released from outside the vehicle. Such apparatus is disclosed in the embodiment illustrated in Figs. 6 through 9.

In the embodiment of the invention illustrated in Figs. 6 through 9, the prisoner 12 is seated within the prisoner compartment of the vehicle which has a conventional door 116 pivotably mounted on a rail or post 118 for closing within the opening of the door frame, the latter including the floor rail 120.

The second strap or belting 36 has a catch member, preferably in the form of a buckle 138 or the like, secured adjacent one end thereof, the belting being connected to the strap 18 at its other end. The buckle 138 has a central rib 140 and one end of the belt 36 may thus be disposed about the central rib, folded back over one surface of the rib and sewn to the major portion of the belt 36 as illustrated at 142. The buckle has a substantially planar rear surface 144 which contact the belt when the buckle pivots. The other end of the belt may either be fixedly or adjustably attached to the strap 18. For example, in Fig. 8 the end of the belt 36 remote from the buckle 138 may be secured about the grommet 146 or other similar ring member by stitching the folded back end portion 48 of the belt 36 in a similar manner to that at the other end. The grommet 146 is not permanently attached to the strap 18 but receives the free end of the strap and is disposed so as to freely move therealong. The belt 36 is of a length sufficient such that the buckle 138 may extend out of the vehicle while the strap 18 is secured about the prisoner within the vehicle. Additionally, the substantially planar surface 144 of the buckle provides a surface which in use abuts the door and door frame and acts as a bearing surface to spread the load so as not to damage the vehicle.

In the use of the apparatus of the second embodiment, after the prisoner is handcuffed, the strap 18 may be applied about his legs or thighs prior to or as he enters the vehicle. The buckle or catch 138 may then be tossed out of the vehicle and the door 116 slammed shut. The catch will then be prevented from being pulled into the prisoner compartment as it becomes lodged against the door and the floor rail 120 or other portions of the door frame, thereby to restrain the prisoner from violently kicking his feet. In the preferred form of the second embodiment of the invention, the catch or buckle may have a magnet 150 of similar shape to the buckle bonded to at least the rear surface 144, i.e., the surface which engages the door and frame. Thus, after the prisoner is in the vehicle and the door shut, the buckle will adhere to the door and frame at the first pulling move of the prisoner as the belt 36 slips in the space between the door and the frame. Once the buckle so adheres to and becomes attached to the metal of the door, it will not slip away from the vehicle as the prisoner relaxes. This prevents the buckle from falling from the vehicle and becoming grasped or entangled by an object when the vehicle is moving.

Numerous alterations of the structure herein disclosed will suggest themselves to those skilled in the art. However, it is to be understood that the present disclosure relates to the preferred embodiment of the invention which is for purposes of illustration only and not to be construed as a limitation of the invention. All such modifications which do not depart from the spirit of the invention are intended to be included within the scope of the appended claims.

**Claims**

1. Apparatus for restraining the lower limbs of a prisoner or the like to a vehicle within which the prisoner is confined, said apparatus comprising an elongated laminated strap having first and second plies of material bonded together, the first ply comprising a plastic sheet having hook and loop fastener elements extending from a surface thereof, the hooks extending from a location on said sheet toward one end and the loops extending from proximate said location toward the other end, said second ply comprising a vinyl sheet of the same size as said first ply, a grommet fastened to one of said ends of said strap, the second of said ends being a free end, said grommet being of a size for receiving said free end of said strap and permitting said free end to be drawn therethrough and folded over said grommet with portions of said hook and loop elements disposed in facing relationship so as to cooperatively adhere together when engaged, whereby said strap may be adjustably disposed about the lower limbs of said prisoner to lock said limbs together, and a belt of finite length having a first end connected to said strap and having securing means fastened at another end for rapid attachment to said vehicle.

2. Apparatus as recited in claim 1, wherein said belt is fixedly attached to said strap.

3. Apparatus as recited in claim 2, including a second grommet fastened to said one of said ends, and said belt is fastened to said second grommet.

4. Apparatus as recited in claim 1, wherein said belt is adjustably disposed on said strap.

5. Apparatus as recited in claim 4, including a second grommet disposed about said strap for movement therealong, and said belt is fastened to said second grommet.

6. Apparatus as recited in claim 1, wherein an anchor is secured to a support surface of said vehicle and said securing means is a hook means having a latch means for rapid attachment to said anchor.

7. Apparatus as recited in claim 6, wherein said anchor is secured to the floor of said vehicle.

8. Apparatus as recited in claim 6, wherein said latch means is resiliently biased to close said hook means.

9. Apparatus as recited in claim 1, wherein said vehicle includes a door closable within a door frame of the vehicle, said belt having a first end connected to said strap and having a catch secured at another end, the length of said belt being sufficient such that the catch may be disposed outside the vehicle when the prisoner is within the vehicle, said catch being of a size not to slip between said door and said frame when the door is shut.

10. Apparatus as recited in claim 9, wherein said catch has a substantially planar surface for bearing against said door and adjacent portions of the door frame when said belt is pulled into the vehicle.

11. Apparatus as recited in claim 10, wherein said substantially planar surface includes a magnet secured thereto for adhering to said door and frame.

FIG. 1

FIG. 3

FIG. 4

FIG. 5

FIG. 2

FIG.6

FIG.7

FIG.8

FIG.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-4 004 583 (JOHNSON) * column 1, line 47 - column 2, line 15 * | 1,2,9 | A 61 F 5/37 B 60 R 22/10 |
| Y | US-A-3 812 852 (KONVALIN) * figure 3; column 2, line 51 - column 3, line 4 * | 1,2,9 | |
| A | GB-A-1 056 714 (OSMOND & OSMOND) * figure 1 * | 1,4-6,8 | |
| A | DE-B-1 815 699 (SCHMIDT) * column 3, lines 21-33; figures 3-5 * | 1,6,8 | |
| A | US-A-2 877 833 (BOLES) * column 2, lines 33-42 * | | |
| A | US-A-4 414 969 (HYMAN) * figure 1 * | | |

TECHNICAL FIELDS SEARCHED (Int. Cl.4)

A 44 B 11/00
A 61 F 5/00
B 60 R 22/00
B 63 H 9/00

The present search report has been drawn up for all claims

| Place of search | Date of completion of the search | Examiner |
|---|---|---|
| BERLIN | 22-04-1988 | STANDRING M A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)